**Europäisches Patentamt**

**European Patent Office**

**Office européen des brevets**

(19)

(11) Veröffentlichungsnummer: **0 491 128 B1**

(12) **EUROPÄISCHE PATENTSCHRIFT**

(45) Veröffentlichungstag der Patentschrift: **22.03.95**

(51) Int. Cl.6: **C07C 31/30**, C07C 29/68, C08F 4/00

(21) Anmeldenummer: **91117612.1**

(22) Anmeldetag: **16.10.91**

(54) **Verfahren zur Herstellung von Erdalkalialkoholaten mit sphärischem Partikelhabitus.**

(30) Priorität: **17.12.90 DE 4040252**

(43) Veröffentlichungstag der Anmeldung:
**24.06.92 Patentblatt 92/26**

(45) Bekanntmachung des Hinweises auf die
Patenterteilung:
**22.03.95 Patentblatt 95/12**

(84) Benannte Vertragsstaaten:
**AT BE DE ES FR GB IT NL SE**

(56) Entgegenhaltungen:
**EP-A- 0 159 150**
**EP-A- 0 216 402**
**EP-A- 0 436 801**
**US-A- 4 806 696**

(73) Patentinhaber: **HÜLS AKTIENGESELLSCHAFT**

**D-45764 Marl (DE)**

(72) Erfinder: **Standke, Burkhart, Dr.**
**Burstelstrasse 2 a**
**W-7888 Rheinfelden (DE)**
Erfinder: **Rauleder, Hartwig, Dr.**
**Uhlandweg 51 a**
**W-7888 Rheinfelden (DE)**
Erfinder: **Biangardi, Harald-Jürgen, Prof. Dr.**
**1428 Chesterfield Estates Drive**
**Chesterfield, MO 63005 (US)**
Erfinder: **Kötzsch, Hans-Joachim, Dr.**
**Fecampring 28**
**W-7888 Rheinfelden (DE)**

## Beschreibung

Die Erfindung betrifft ein Verfahren zur Herstellung von Erdalkalialkoholaten mit sphärischem bzw. sphäroidischem Partikelhabitus im Bereich der Partikelgrößen 1 bis 500 $\mu$m durch Fällung aus homogener Lösung.

Bei der technischen Synthese von Erdalkalialkoholaten z.B. aus Erdalkalimetallspänen und Alkohol fällt das entsprechende Alkoholat in unregelmäßiger Kornstruktur an. Viele Anwendungen verlangen jedoch speziell konfektionierte Produkte; z.B. sphärische Partikel mit relativ monomodaler Kornverteilung. Für die Polyolefinsynthese mit Ziegler-Katalysatoren haben sich aktive Katalysatorträgersubstanzen auf Basis Magnesiumethylat mit sphärischem Partikelhabitus und einem mittleren Korndurchmesser von 10 bis 20 $\mu$m besonders bewährt (EP-OS 0 236 082, 0 159 150). Die Verfahren zur Herstellung von sphärischen bzw. sphäroidischen Erdalkalialkoholaten erweisen sich als technisch aufwendig; die Sprühtrocknung carbonisierter ethanolischer Magnesiumethylatlösungen mit anschließender Decarbonisierung durch Tempern im Stickstoffstrom (EP-OS 0 236 082) liefert zwar ein Magnesiumethylatpulver mit angenähert kugeligem Partikelhabitus, jedoch mit stark strukturierter Oberfläche, resultierend aus dem Kollabieren von beim Sprühvorgang entstehenden Hohlkugeln (Rosinenstruktur). Da die Sprühtrocknung unter Inertgasbedingungen gefahren werden muß, ist das verfahren technisch sehr aufwendig, wenn das Lösemittel aus dem großen für die Sprühtrocknung benötigten Inertgasstrom recyliert werden soll. Partikelgrößen <10 $\mu$m sind in technischen Sprühanlagen nach diesem Verfahren nicht zugänglich ebenso wie durch Fällungsreaktionen nach EP-OS 0 241 891 in komplexen Lösemittelgemischen.

Gemäß der vorliegenden Erfindung wird erreicht, auf Basis einer homogenen Fällungsreaktion, Erdalkalialkoholate der allgemeinen Zusammensetzung (1) mit sphärischem oder sphäroidischem Partikelhabitus im Korngrößenbereich von 0,5 bis 500 $\mu$m herzustellen.

$$M (OR^1)_a (OR^2)_b (CO_2)_x \qquad (1)$$

M = Metall aus der Gruppe II des Periodensystems der chemischen Elemente;

$R^1$, $R^2$ = Alkylrest, wobei gegebenenfalls $R^1$ = $R^2$ sein kann,

$a + b = 2$, $0 \leq a \leq 2$, $0 \leq b \leq 2$, $0 \leq x \leq 2$.

$R^1$ und $R^2$ sind Alkylreste mit 1 bis 8, vorzugsweise 1 bis 6 C-Atomen.

Das Verfahren basiert auf folgender allgemeiner Vorgehensweise:

1. Lösen von Erdalkalialkoholaten in einem geeigneten Lösemittel $L_1$, gegebenenfalls durch zusätzliche Wirkung von $CO_2$ als Lösevermittler.
2. Mischen der in 1. erhaltenen homogenen Lösung mit einem Lösemittel $L_2$, in welchem das reine Erdalkalialkoholat oder eine auftretende Verbindung des Erdalkalialkoholats mit dem Lösevermittler unlöslich oder nur schwer löslich ist, das jedoch mischbar ist mit dem in 1. zur Lösung des Erdalkalialkoholats verwendeten Lösemittel $L_1$, sowie die destillative Trennung der in 1. und 2. verwendeten Lösemittel $L_1$ und $L_2$ in einer Weise ermöglicht, daß nach destillativer Entfernung des in 1. verwendeten Lösemittels $L_1$ aus der in 2. erhaltenen homogenen Mischung ein Fällungsprodukt mit der Zusammensetzung (1) dispergiert in Lösemittel $L_2$ zurückbleibt.
3. Abdestillieren von $L_1$. Dabei erfolgt die Bildung von Partikeln mit der Zusammensetzung (1), dispergiert in $L_2$.
4. Trennung des Fällungsproduktes von $L_2$: a) Filtration und anschließende Trocknung des Filterkuchens; b) Abdestillieren von $L_2$ vom Fällungsprodukt.

Die Steuerung der Partikelgröße kann in Verfahrensschritt 3 erfolgen:

a) Steuerung der Partikelgröße über das Schergefälle: Bei geringem Schergefälle (beispielsweise Verwendung von Flügelrührern mit geringer Drehzahl) werden rundliche Partikel im Größenbereich bis zu 500 $\mu$m erhalten. Durch Verwendung von "high shear" Dispergierwerkzeugen nach dem Rotor-Stator-Prinzip (z.B. Ultra-Turrax, Fa. Jahnke und Kunkel) kann die Partikelgröße auf bis zu 2 bis 3 $\mu$m erniedrigt werden. Je nach Schergefälle (abhängig vom Dispergierwerkzeug und der eingestellten Umfangsgeschwindigkeit) sind auch Zwischengrößen erhältlich.

b) Tröpfchenstabilisierung durch Verwendung von Emulgierhilfsstoffen: Da im Verfahrensschritt 3 bei der destillativen Entfernung von $L_1$ aus dem homogenen Reaktionsgemisch vor der Bildung harter Partikel zunächst eine Emulsion entsteht, bestehend aus $L_2$ als kontinuierliche Phase und einer homogenen viskosen Phase, zusammengesetzt aus $L_1$, $L_2$ und der Erdalkaliverbindung der Zusammensetzung (1) als emulgierte Phase, können die durch das Schergefälle erzeugten Tröpfchen der emulgierten Phase mittels geeigneter Emulsionsstabilisatoren, welche sich an der Grenzfläche zwischen kontinuierlicher und emulgierter Phase ansiedeln, stabilisiert werden. Die Stabilisierung der in Verfahrensschritt 3. zunächst gebildeten Emulsion durch Hilfsstoffe fördert die Herstellung vorzugsweise kleiner Partikel (Durchmesser 0,5 bis 20 $\mu$m) mit nahezu monomodaler Kornverteilung, d.h. Partikel mit enger Verteilung der Korngrößen und sphärischem Partikelhabitus. Als Emulsionsstabilisator eignet sich bei dem beschriebenen Verfahren insbesondere pyrogene Kieselsäure (z.B. Cab-O-Sil TS 720, Cab-O-Sil M5). Bei pyrogener Kieselsäure sind die Anforderungen an einen geeigneten Feststoff-Emulsionsstabilisator erfüllt: genügende Dispersität (Primärpartikelgrößen im Bereich weniger Nanometer, leicht dispergierbar in $L_1$ und $L_2$) und nicht vollständige Benetzbarkeit des Feststoffes (Kieselsäure) durch die innere flüssige Phase (emulgierte Phase), bestehend aus der Erdalkaliverbindung mit Zusammensetzung (1), in den Lösungsmitteln $L_1$ und $L_2$.

Untersuchungen im Transmissionselektronenmikroskop und Untersuchungen an Ultradünnschnitten (150 nm Dicke) zeigen, daß derartig stabilisierte Fällungspartikel $SiO_2$-beschichtete Erdalkalialkoholat-Kugeln darstellen. Die Dicke der Kieselsäureschicht beträgt 40 bis 50 nm. Durch Dispergierung derartiger Partikel in einem inerten Lösemittel und Behandlung der Dispersion mit Ultraschall oder "high-shear"-Dispergierer läßt sich die Kieselsäurebeschichtung weitgehend von der Partikeloberfläche lösen. Die Trennung von Erdalkalialkoholat und Kieselsäure erfolgt im Anschluß über Sedimentation.

Durch Zusammenwirken der Verfahrensschritte 1 bis 3 wird die Aufgabe gelöst, sphärische bzw. sphäroidische Partikel von Erdalkalialkoholaten mit der Zusammensetzung (1) herzustellen. Der Begriff "sphärisch" bedeutet kugeliger Habitus, während mit sphäroidisch ein annähernd kugeliger Kornhabitus umschrieben wird.

Die Anwendung eines kontrollierten, kontinuierlich einwirkenden Schergefälles in Verfahrensschritt 3 führt zu kontinuierlicher Tröpfchenzerteilung innerhalb der sich intermediär bildenden Emulsion, insbesondere von Tröpfchen, die aufgrund von Koaleszenzvorgängen eine gewisse Größe überschreiten. Die Größe der erhaltenen Emulsionströpfchen verhält sich in erster Näherung umgekehrt proportional zum Schergefälle (ein hohes Schergefälle erzeugt kleine Tröpfchen). Geringes Schergefälle erreicht man mit zahlreichen Rührern konventioneller Bauart, während hohe Scherkräfte besonders durch Rotor-Stator-Konstruktionen mit außen liegenden zylindrischen Stator und regelbaren Rotor von wählbarer Gestalt erreichbar ist, deren Umfangsgeschwindigkeiten zwischen 5 und 25 m/s, vorzugsweise zwischen 10 und 21 m/s liegen sollen.

Da im Laufe der destillativen Entfernung des Lösemittels $L_1$ die Viskosität der inneren Phase sehr stark ansteigt (bei vollständiger Entfernung von $L_1$ ist die innere Phase als Festkörper zu betrachten), spielen Koaleszensvorgänge am Ende des Verfahrensschrittes 2 keine Rolle mehr, so daß die Abtrennung des Feststoffes vom Lösemittel $L_2$ ohne Einwirkung eines Schergefälles durch Filtration oder durch destillative Entfernung von $L_2$ erfolgen kann.

Auf das kontinuierlich einwirkende hohe Schergefälle kann bei der Herstellung kleiner Partikel im Bereich 0,5 bis 20 $\mu$m verzichtet werden, die eine Koaleszenz der durch Schereinwirkung gebildeten Emulsionströpfchen verhindern. Man erhält auf einfache Weise, reproduzierbar, kompakte, sphärische Partikel mit nahezu monomodaler Kornverteilung.

Die Temperaturen des Verfahrens sind nicht von wesentlichen Einfluß auf Habitus und Größe der Partikel. Zweckmäßig wird Jedoch die Lösung aus Verfahrensschritt 1 und deren Mischung mit $L_2$ bei erhöhter Temperatur hergestellt, soweit die jeweilige Lösung dann eine höhere Konzentration besitzt. Die Temperatur bei der Destillation richtet sich nach dem Siedepunkt des jeweiligen Lösemittels, der in üblicher Weise durch verminderten Druck gesenkt werden kann.

Erdalkalimetall bedeutet sehr bevorzugt Mg, gegebenenfalls auch Ca, Sr oder Ba.

Die Lösemittel $L_1$ sind polar protische Lösemittel, besonders Alkohole mit 1 bis 6 C-Atomen (unverzweigt oder verzweigt), vorzugsweise Methanol bis Butanol. Verwendet werden können ebenfalls Gemische der besagten Lösemittel.

Die Menge der Lösemittel $L_1$ richtet sich nach der Löslichkeit des Alkoholats im Lösemittel $L_1$ bzw. des carbonisierten Alkoholats im Lösemittel $L_1$, wobei sehr bevorzugt eine gesättigte Lösung des Alkoholats verwendet wird.

Die Lösemittel $L_2$ sind polar oder unpolar aprotische Lösemittel, insbesondere Kohlenwasserstoffe (unverzweigt, verzweigt, aromatisch oder Gemische hiervon), Ether oder Tetraalkoxysilane, gegebenenfalls auch Ketone, Carbonsäureester oder Alkylalkoxysilane mit einem Siedepunkt höher als $L_1$ und einer geringeren Löslichkeit des Erdalkalialkoholats in $L_2$ als in $L_1$. Verwendet werden können ebenfalls Gemische der aufgeführten Lösemittel $L_2$.

Die Menge des Lösemittels $L_2$ kann in weiten Grenzen variieren, da in jedem Fall eine Fällung eintritt in dem Maße wie $L_1$ entfernt wird. Bevorzugt wird mit Feststoffkonzentrationen am Ende des Verfahrensschrittes 3 von y% gearbeitet;

$0 < y \leq 70$, vorzugsweise $1 \leq y \leq 10$.

Verwendet man zur Lösung des Erdalkaliakoholats einen anderen Alkohol als denjenigen, der dem Alkoholatrest des zu lösenden Erdalkalialkoholats entspricht, oder verwendet man als $L_1$ oder $L_2$

Lösemittel, die im Laufe der Reaktion derartige Alkohole abspalten können - z.B. Carbonsäureester, Kieselester, Titanester etc. - so tritt partiell Umalkoholisierung an der Erdalkaliverbindung ein.

Man isoliert dann Mischalkoholate der allgemeinen Zusammensetzung (1).

Eine Basisanwendung speziell konfektionierter Erdalkalialkoholate ist die Verwendung als aktives Katalysatorträgermaterial bei der Polyolefinsynthese. Die Rezepturen zur Herstellung hochaktiver Katalysatoren - z.B. für die Propenpolymerisation - auf Basis Magnesiumalkoholat als aktives Katalysatorträgermaterial - sind bekannt. Da Partikelmorphologie und Breite der Verteilung bei dem erfindungsgemäßen Verfahren den Stand der Technik (z.B. Sprühtrocknung carbonisierter Magnesiumalkoholatlösungen) überlegen sind, wirkt sich dieses günstig auf die Eigenschaften des Polymerisatpulvers aus. Bei der isotaktischen Propenpolymerisation ist die Polymerisatkornstruktur ein genaues, jedoch volumetrisch vergrößertes Abbild der Katalysatorkornstruktur. Verwendung eines nach dem erfindungsgemäßen Verfahren hergestellten Magnesiumethylats als aktives Katalysatorträgermaterial bei der isotaktischen Propenpolymerisation führt zu einer engeren Kornverteilung im Polymerisat - unerwünschtes Über- und Unterkorn wird vermindert - sowie zu einer glatteren Partikeloberfläche.

**Beispiel 1**

Fällung aus homogenem Medium unter Verwendung eines geringen Schergefälles:
In einem Rührreaktor, ausgestattet mit Gaseinleitungsvorrichtung und Destillationsbrücke wird unter trockener Stickstoffatmosphäre 1836 g Ethanol und 292 g Magnesiumethylat (Normalkorn, Handelsprodukt HÜLS AG) gegeben. Unter Rühren werden innerhalb von 2h 194 g $CO_2$ eingeleitet. Es entsteht eine fleichfarbene Lösung von carbonisiertem Magnesiumethylat in Ethanol. Diese Lösung wird mit 2829 g Diethylenglykoldimethylether versetzt. Im Reaktionsgefäß befindet sich zu diesem Zeitpunkt eine homogene Lösung. Bei von 86°C auf 130°C steigender Sumpftemperatur wird innerhalb von 2h die gesamte Ethanolmenge unter Rühren mit einem Flügelrührer abdestilliert. Dabei entsteht eine milchig weiße Trübung (Fällung aus homogenem Medium). Unter trockenen Stickstoff wird anschließend über eine G4-Fritte abgenutscht und mehrmals mit Aceton gewaschen. Das Filtrat besteht aus Diethylenglykoldimethylether. Aus dem Filterkuchen lassen sich nach Trocknung bei 90°C (p<1 mbar) 210g Produkt (festes, carbonisiertes Magnesiumethylat) isolieren. Man erhält Partikel mit 50 bis 200 μm Durchmesser. Das Produkt (Gemisch aus Magnesiumethylat und carbonisiertem Magnesiumethylat) enthält ca. 14% $CO_2$. Der $CO_2$-Gehalt kann auf unter 2% gesenkt werden, indem man 2h bei 180°C (p<1 mbar) tempert.

**Beispiel 2**

Fällung aus homogenem Medium unter Verwendung eines mittleren Schergefälles:
In einen Rührreaktor (Rührer = Ultra-Turrax T 50, Fa. Jahnke und Kunkel), ausgestattet mit Gaseinleitungsvorrichtung und Destillationsbrücke werden unter trockener Stickstoffatmosphäre 1896 g Ethanol und 292 g Magnesiumethylat (Normalkorn, Handelsprodukt Hüls AG) gegeben. Unter Rühren werden innerhalb von 2h 194 g $CO_2$ eingeleitet. Es entsteht eine fleichfarbene Lösung von carbonisiertem Magnesiumethylat in Ethanol. Zu der homogenen Lösung werden unter Rühren 5803 g Xylol gegeben. Anschließend destilliert man das Ethanol innerhalb von 3h bei einer Drehzahl des Dispergierers von 4000 U/min. vollständig ab (maximale Sumpftemperatur 90°C, minimaler Druck 200 mbar). Es entsteht eine Dispersion, die in einem Rotationsverdampfer von Xylol befreit wird. Das zurückbleibende fließfähige weiße Pulver wird anschließend bei einer Temperatur von 180°C (bei einem Druck von weniger als 1 mbar) 2h am Rotationsverdampfer getrocknet. Isoliert werden 233 g Magnesiumethylat ($CO_2$-Gehalt < 2%). Die Korngrößenanalyse zeigt abgerundete Partikel mit einem mittleren Durchmesser von ca. 20 μm.

**Beispiel 3**

Fällung aus homogenem Medium unter Verwendung eines hohen Schergefälles:
Die Versuchsdurchführung entspricht Beispiel 2 mit dem Unterschied, daß die Drehzahl des Dispergiergerätes auf maximale Leistung (10.000 U/min.) geschaltet wird.
Man isoliert nach Trocknung in Rotationsverdampfer 230 g fließfähiges Magnesiumethylat ($CO_2$-Gehalt < 2%).
Die Kornanalyse zeigt abgerundete Partikel mit einem mittleren Durchmesser von ca. 3 μm.

**Beispiel 4**

Fällung aus homogenem Medium, Partikelstabilisierung durch hydrophobierte, pyrogene Kieselsäure:
In einen Rührreaktor, ausgestattet mit Gaseinleitungsvorrichtung und Destillationsbrücke wird unter trockener Stickstoffatmosphäre 1836 g Ethanol und 292 g Magnesiumethylat (Normalkorn, Handelsprodukt Hüls AG) gegeben. Unter Rühren werden innerhalb von 2h 194 g $CO_2$ eingeleitet. Es entsteht eine fleischfarbene Lösung von carbonisiertem Ma-

gnesiumethylat in Ethanol. In einem zweiten Kolben werden 173 g hydrophobierte, pyrogene Kieselsäure (Cab-O-Sil TS 720) in 5803 g Xylol mittels Ultra-Turrax (T 50, Fa. Jahnke und Kunkel) zu einer leicht opaleszierenden Suspension dispergiert. Die Kieselsäuredispersion wird anschließend unter Rühren in die Lösung von carbonisiertem Magnesiumethylat gegeben. Innerhalb von 6h destilliert man unter Normaldruck sämtliches Ethanol ab (maximale Sumpftemperatur 125° C). Dabei trübt sich die anfangs nur leicht opaleszierende Flüssigkeit deutlich. Die entstandene Dispersion wird in einen Rotationsverdampfer unter Abtrennung von Xylol zur völligen Trockene gebracht ($T_{max}w$ = 180° C, $p_{min.}$ <1 mbar). Es entstehen 380 g eines fließfähigen, feinteiligen Magnesiumethylatpulvers mit einem $CO_2$-Gehalt < 2%. Die Licht- und Elektronenmikroskopische Analyse zeigt, daß das entstandene Fällungsprodukt aus sphärischen in Mittel ca. 5 $\mu$m messenden Magnesiumethylat-Partikeln besteht, die eine ca. 50 nm starke Kieselsäurebeschichtung aufweisen.

## Beispiel 5

Fällung aus homogenem Medium, Partikelstabilisierung durch hydrophile, pyrogene Kieselsäure: In einen Rührreaktor, ausgestattet mit Gaseinleitungsvorrichtung und Destillationsbrücke wird unter trockener Stickstoffatmosphäre 1836 g Ethanol und 292 Magnesiumethylat (Normalkorn, Handelsprodukt Hüls AG) gegeben. Unter Rühren werden innerhalb von 2 h 194 g $CO_2$ eingeleitet. Es entsteht eine fleischfarbene Lösung von carbonisiertem Magnesiumethylat in Ethanol.

In einem zweiten Kolben werden 214 g pyrogene Kieselsäure (Cab-O-Sil M5) in 3571 g n-Heptan mittels Ultra-Turrax (T 50, Fa. Jahnke u. Kunkel) zu einer leicht opaleszierenden Suspension dispergiert. Die Kieselsäuredispersion wird anschließend unter Rühren in die Lösung von carbonisiertem Magnesiumethylat gegeben. Innerhalb von 3h destilliert man unter Normaldruck sämtliches Ethanol (im Azeotrop mit n-Heptan) ab (von 83° C auf 89° C steigende Sumpftemperatur). Dabei trübt sich die anfangs nur leicht opaleszierende Flüssigkeit deutlich. Die entstandene Dispersion wird in einen Rotationsverdampfer unter Abtrennung von restlichem n-Heptan zur völligen Trockene gebracht ($T_{max.}$ = 180° C, $P_{min.}$ <1 mbar). Es entstehen 380g eines fließfähigen, feinteiligen Magnesiumethylatpulvers mit einen $CO_2$-Gehalt <2%. Licht- und Elektronenmikroskopische Analyse zeigt, daß das entstandene Fällungsprodukt aus sphärischen ca. 1 $\mu$m messenden Magnesiumethylat-Partikeln besteht, die durch Kieselsäure beschichtet sind.

## Beispiel 6

Fällung von Magnesiumethylat-methylat-Mischalkoholat.

In einem heizbaren 21-Kolben ausgerüsteten mit Ultra-Turrax T50 (Fa. Jahnke u. Kunkel, Rührfrequenz 4.000 U/min.) und 30 cm Destillationskolonne werden 500 ml Magnesiummethylat-Lösung (hergestellt durch Lösen von 18 g Magnesiummetall in 500 ml Methanol) mit 763.6 g Tetraethoxysilan versetzt. Anschließend destilliert man bei einer Sumpftemperatur von Raumtemperatur auf 150° C steigend ein Gemisch von Methanol, Ethanol, Tetraethoxysilan, Triethoxymethoxysilan, Diethoxydimethoxysilan, Ethoxytrimethoxysilan und Tetramethoxysilan ab (Zusammensetzung -GC Flächenprozent-: 37% Methanol, 40% Ethanol, 23% Silane). Die verbleibende Dispersion wird unter Schutzgas in den Kolben eines Rotationsverdampfers verbracht und bei einem Druck von Umgebungsdruck auf <1 mbar sinkend sowie einer maximalen Temperatur von 170° C zur Trockene eingeengt. Man erhält 38g eines fließfähigen Magnesiumalkoholatpulvers der Zusammensetzung Mg $(OC_2H_5)_{1,22}$-$(CH_3)_{0,78}$ mit einer mittleren Korngröße von ca. 10 $\mu$m (lichtmikroskopische Auswertung).

## Beispiel 7

Fällung von Magnesiummethlyat:
In einen heizbaren 21-Kolben, ausgerüstet mit Flügelrührer und 30 cm Destillationskolonne, werden 500 ml Magnesiummethylatlösung (hergestellt durch Lösen von 18 g Magnesiummetall in 500 ml Methanol) mit 750 g Tetramethoxysilan versetzt. Anschließend destilliert man bei einer Sumpftemperatur von Raumtemperatur auf 150° C steigend ein Gemisch von Methanol und Tetramethoxysilan ab (Zusammensetzung -GC Flächenprozent-: 55% Methanol, 45 % Tetramethoxysilan). Die verbleibende Dispersion wird unter Schutzgas in den Kolben eines Rotationsverdampfers verbracht und bei einem Druck von Umgebungsdruck auf <1 mbar sinkend sowie einer maximalen Temperatur von 170° C zur Trockene eingeengt. Man erhält 30g eines fließfähigen Magnesiummethylatpulvers mit einer mittleren Korngröße von ca. 50 $\mu$m (lichtmikroskopische Auswertung).

## Beispiel 8

Anwendung als Katalysatorträgers für die Propenpolymerisation.
20 g des aus Beispiel 4 erhaltenen Magnesiumethylats werden unter trockener Stickstoffatmosphäre mittels "high shear"-Dispergierer in 200 ml absoluten Ethanol dispergiert. Durch die Einwirkung der hierbei auftretenden Scherkräfte wird die Kieselsäurebeschichtung zum größten Teil gelöst. Nach zweistündiger Sedimentation wird die überstehende, leicht trübe, opaleszierende Phase abdekantiert. Diese Phase enthält den Hauptteil der Kieselsäure. Das Sediment, bestehend aus sphärischem Magnesiumethylat (mittlerer Partikeldurchmesser 5 $\mu$m) wird destillativ von Ethanol befreit und nach bekanntem Verfahren (EP 0 236 082) zu einem für die Propenpolymerisation geeigneten Katalysator umgesetzt. Der Katalysator erweist sich bei der Propenpolymerisation bezüglich Aktivität und Stereospezifität als dem Stand der Technik entsprechend.

Die Polymerisatpartikel besitzen wie die Katalysatorträgerpartikel nahezu exakte Kugelgestalt mit glatter Oberfläche. Die Korngrößenanalyse (Lasermessung) zeigt eine enge Kornverteilung: bei einem mittleren Partikeldurchmesser von 152 $\mu$m beträgt der Anteil an Partikeln > 1000 $\mu$m 0,1% (Überkorn) und der Anteil an Partikeln < 100 $\mu$m 5,8%.

**Beispiel 9**

Vergleichsbeispiel:
Sprühtrocknung carbonisierter Magnesiumethylatlösung und Anwendung als Katalysatorträger für die Propenpolymerisation; nicht Patentgegenstand.
In einen Rührreaktor, ausgestattet mit Gaseinleitungsvorrichtung und Destillationsbrücke wird unter trockener Stickstoffatmosphäre 1836 g Ethanol und 292 g Magnesiumethylat (Normalkorn, Handelsprodukt Hüls AG) gegeben. Unter Rühren werden innerhalb von 2h 194 g $CO_2$ eingeleitet. Es entsteht eine fleischfarbene Lösung von carbonisiertem Magnesiumethylat in Ethanol. In einem Laborsprühtrockner (Fa. Büchi) wird diese Lösung bei einen Durchsatz von 307 ml/h, einer Temperatur von 190°C an der Sprühdüse und einm Stickstoffdurchsatz von ca. 600 l/h einer Sprühtrocknung unterzogen. Das isolierte, fließfähige Pulver besteht aus carbonisiertem Magnesiumethylat. Zur Decarbonisierung und zur Entfernung der Restfeuchte (Ethanol) wird das Pulver 2 h bei 180°C und einem Druck < 1 mbar getempert. Der mittlere Korndurchmesser beträgt ca. 5 $\mu$m bei einer stark strukturierten Oberfläche (Rosinenstruktur). Nach einem bekannten Verfahren (EP 0 236 082) wird das Pulver zu einem für die Propenpolymerisation geeigneten Katalysator umgesetzt. Der Katalysator erweist sich bei der Propenpolymerisation bezüglich Aktivität und Stereospezifität als dem Stand der Technik entsprechend. Die Polymerisatpartikel besitzen wie die Katalysatorpartikel eine sehr unregelmäßige Oberflächenstruktur. Die Korngrößenanalyse (Lasermessung, Siebanalyse) zeigt eine relativ breite Kornverteilung: bei einem mittleren Partikeldurchmesser von 140 $\mu$m beträgt der Anteil an Partikeln

> 1000 $\mu$m 4,7% und der Anteil an Partikeln <100 $\mu$m 23,5%. Der Anteil an Über- und Unterkorn ist damit bei vergleichbarer mittlerer Korngröße sowohl des Katalysatorträgerkorns als auch der Polymerisatpartikel beim Sprühtrocknungsverfahren erheblich größer als bei dem erfindungsgemäßen Fällverfahren.

**Patentansprüche**

1.  Verfahren zur Herstellung von Erdalkalimetallverbindungen,

    $$M\,(OR^1)_a\,(OR^2)_b\,(CO_2)_x \qquad (1)$$

    M =
    Metall aus der Hauptgruppe II des Periodensystems der chemischen Elemente;
    $R^1$ und $R^2$ =
    gleiche oder verschiedene Alkyl-Radikale mit 1 bis 6 Kohlenstoffatomen;
    $a + b = 2,\ 0 \leq a \leq 2,\ 0 \leq b \leq 2,$
    $0 \leq x \leq 2$
    mit sphärischem bzw. sphäroidischem Partikelhabitus, dadurch gekennzeichnet, daß die Partikel aus einer homogenen Lösung, bestehend aus einem Erdalkalialkoholat oder seiner carbonisierten Form, gelöst in einem Lösemittel $L_1$, versetzt mit einem zweiten Lösemittel $L_2$, welches die Eigenschaft besitzt mit der Lösung des Erdalkalialkoholats mischbar zu sein, jedoch das Erdalkalialkoholat oder seine carbonisierte Form nicht oder nur schwer löst, durch Abdestillieren des Lösemittels $L_1$, entstehen.

2.  Verfahren nach Anspruch 1, dadurch gekennzeichnet, daß $L_1$ ein polar protisches und $L_2$ ein polar aprotisches oder unpolar aprotisches Lösemittel darstellt.

3.  Verfahren nach Anspruch 1 oder 2, dadurch gekennzeichnet, daß $L_1$ ein unverzweigter oder verzweigter Alkohol mit 1 bis 6 C-Atomen ist.

4.  Verfahren nach Anspruch 1 oder 2, dadurch gekennzeichnet, daß $L_2$ ein Ether, besonders ein Ether des Ethylenglykols, ein aliphatischer, cyclischer, aromatischer Kohlenwasserstoff, ein Chlorierungsprodukt dieser Kohlenwasserstoffe, ein Tetraalkoxysilan, besonders Tetraethoxy- oder Tetramethoxysilan, ein Alkyl-, Dialkyl- oder Trialkylalkoxysilan darstellt.

5.  Verfahren nach einem der Ansprüche 1 bis 4, dadurch gekennzeichnet, daß $L_1$ Ethanol, Methanol, Propanol, Isopropanol oder Butanol und als $L_2$ Xylol, Diethylenglykoldimethylether, Tetraethoxysilan oder Tetramethoxysilan verwendet wird.

6. Verfahren nach einem der vorgenannten Ansprüche, dadurch gekennzeichnet, daß als Erdalkalialkoholat Magnesiummethylat oder Magnesiumethylat verwendet wird.

7. Verfahren nach Anspruch 6, dadurch gekennzeichnet, daß zur Erhöhung der Löslichkeit des Erdalkalialkoholats im Lösemittel $L_1$ $CO_2$ verwendet wird.

8. Verfahren nach Anspruch 7, dadurch gekennzeichnet, daß die Partikelgrößensteuerung über Kontrolle des bei der Fällung angewandten Schergefälles oder durch Partikelstabilisierung mittels Zusatz pyrogener Kieselsäure während der Fällungsreaktion vorgenommen wird.

9. Verfahren nach mindestens einem der Ansprüche 1 bis 5, dadurch gekennzeichnet, daß sphärische oder sphäroidische Partikel auf Basis von Erdalkalialkoholaten, hergestellt nach Anspruch 1 bis 8, als aktive Katalysatorträgermaterialien bei der Polyolefinsynthese verwendet werden.

10. Verfahren nach Anspruch 9, dadurch gekennzeichnet, daß die Erdalkalialkoholate Magnesiumalkoholate sind.

## Claims

1. Method for the preparation of alkaline earth metal compounds,

$$M\,(OR^1)_a\,(OR^2)_b\,(CO_2)_x \qquad (1)$$

M =
Metal from the main group II of the Periodic System of the Chemical Elements;
$R^1$ and $R^2$ =
the same or different alkyl radicals with one to six carbon atoms;
$a + b = 2$, $0 \leq a \leq 2$, $0 \leq b \leq 2$,
$0 \leq x \leq 2$
with spherical or spheroidal particle physical appearance, characterised in that the particles are obtained from a homogeneous solution consisting of an alkaline earth alcoholate or its carboxylated form, dissolved in a solvent $L_1$, treated with a second solvent $L_2$, which possesses the property of being miscible with the solution of the alkaline earth alcoholate, but with the alkaline earth alcoholate or its carboxylated form not being dissolved or only being dissolved with difficulty, by distillation of the solvent $L_1$.

2. Method according to claim 1, characterised in that $L_1$ represents a polar protic solvent and $L_2$ represents a polar aprotic or non-polar aprotic solvent.

3. Method according to claim 1 or 2, characterised in that $L_1$ is an unbranched or branched alcohol with 1 to 6 C-atoms.

4. Method according to claim 1 or 2, characterised in that $L_2$ represents an ether, in particular an ether of ethyleneglycol, an aliphatic, cyclic, aromatic hydrocarbon, a chlorination product of this hydrocarbon, a tetraalkoxysilane, especially tetraethoxy- or tetramethoxysilane, an alkyl-, dialkyl- or trialkylalkoxysilane.

5. Method according to one of claims 1 to 4, characterised in that ethanol, methanol, propanol, isopropanol or butanol is used for $L_1$ and xylene, diethyleneglycoldimethylether, tetraethoxysilane or tetramethoxysilane is used as $L_2$.

6. Method according to one of the foregoing claims, characterised in that magnesium methylate or magnesium ethylate is used as alkaline earth alcoholate.

7. Method according to claim 6, characterised in that $CO_2$ is used to increase the solubility of the alkaline earth alcoholate in the solvent $L_1$.

8. Method according to claim 7, characterised in that the particle size control is provided by control of the shear gradient used in the deposition or by particle stabilization by means of pyrogeneous silicic acid during the precipitation reaction.

9. Method according to at least one of claims 1 to 5, characterised in that the spherical or spheroidal particles based on alkaline earth alcoholates produced according to claims 1 to 8, are used as active catalyst materials in polyolefin synthesis.

10. Method according to claim 9, characterized in that the alkaline earth alcoholates are magnesium alcoholates.

## Revendications

1. Procédé de prépararion de composés de métaux alcalino terreux :

$$M\,(OR^1)_a\,(OR^2)_b\,(CO_2)_x \qquad (1)$$

dans lesquels M représente un métal du groupe principal II du système ou Tableau Périodique des Eléments chimiques;

$R^1$ et $R^2$ représentent des radicaux alkyles identiques ou différents ayant 1 à 6 atomes de carbone ;

la somme (a + b) vaut 2 ; $0 \leq a \leq 2$; $0 \leq b \leq 2$; $0 \leq x \leq 2$,

ayant un habitus ou une forme sphérique ou sphéroïdale de particules, procédé caractérisé en ce que les particules sont obtenues à partir d'une première solution, homogène, consistant en un alcoolate alcalino terreux ou sa forme carbonatée, en dissolution dans un solvant $L_1$, additionnée d'un second solvant $L_2$, qui possède la propriété d'être miscible avec la solution de l'alcoolate alcalino terreux mais de ne pas dissoudre ou de ne dissoudre que difficilement l'alcoolate alcalino terreux ou sa forme carbonatée, par le départ par distillation du solvant $L_1$.

2. Procédé selon la revendication 1, caractérisé en ce que $L_1$ représente un solvant protique polaire et $L_2$ un solvant aprotique polaire ou un solvant aprotique non polaire.

3. Procédé selon la revendication 1 ou 2, caractérisé en ce que $L_1$ est un alcool non ramifié ou ramifié comportant 1 à 6 atomes de carbone.

4. Procédé selon la revendication 1 ou 2, caractérisé en ce que $L_2$ représente un éther, en particulier un éther de l'éthylène glycol, un hydrocarbure aliphatique, cyclique ou aromatique, un produit de chloration de ces hydrocarbures, un tétralcoxy silane, en particulier le tétraéthoxysilane ou le tétraméthoxysilane, un alkyl-, dialkyl- ou trialkylalcoxysilane.

5. Procédé selon l'une des revendications 1 à 4, caractérisé en ce qu'on utilise pour $L_1$ l'éthanol, le méthanol, le propanol, l'isopropanol ou le butanol et en ce qu'on utilise comme $L_2$ le xylène, l'éther diméthylique du diéthylène glycol, le tétraéthoxysilane ou le tétraméthoxysilane.

6. Procédé selon l'une des revendications précédentes, caractérisé en ce qu'on utilise comme alcoolate alcalino terreux le méthylate de magnésium ou l'éthylate de magnésium.

7. Procédé selon la revendication 6, caractérisé en ce qu'on utilise $CO_2$ pour augmenter la solubilité de l'alcoolate alcalino terreux dans le solvant $L_1$.

8. Procédé selon la revendication 7, caractérisé en ce qu'on effectue le réglage de la grosseur des particules en réglant, pendant la réaction de précipitation, le gradient du cisaillement appliqué lors de la précipitation ou en stabilisant les particules par l'addition d'acide silicique (silice) de pyrogénation.

9. Procédé selon l'une au moins des revendications 1 à 5, caractérisé en ce qu'on utilise des particules sphériques ou sphéroïdales, à base d'alcoolates alcalino terreux, préparées selon l'une des revendications 1 à 8, comme matière active de support de catalyseur dans la synthèse de polyoléfines.

10. Procédé selon la revendication 9, caractérisé en ce que les alcoolates alcalino terreux sont des alcoolates de magnésium.